# EUROPEAN PATENT APPLICATION

(11) **EP 4 342 894 A1**
(43) Date of publication of application: **27.03.2024**
(21) Application number: 22833713.5
(22) Date of filing: 01.07.2022
(51) Int. Cl.: C07D 403/04, A61K 31/4155

(54) **METHOD FOR PREPARING XANTHINE OXIDASE INHIBITOR**

(30) Priority: 02.07.2021 KR 20210087049
(71) Applicant: LG Chem, Ltd., Seoul 07336 (KR)
(72) Inventor: LEE, Seok Ju, Seoul 07796 (KR); JEONG, Hui Rak, Seoul 07796 (KR); HAM, Jin Ok, Seoul 07796 (KR); SHIN, Doo Sup, Seoul 07796 (KR)
(74) Representative: Hoffmann Eitle
(86) International application number: PCT/KR2022/009549
(87) International publication number: WO 2023/277660

(57) **Abstract**

The present invention relates to a method for preparing a xanthine oxidase inhibitor and, more specifically, to a method for preparing a compound of chemical formula 2 by using ester hydrolysis and a recrystallization method.

## Description

### TECHNICAL FIELD

The present invention relates to a method for preparing a xanthine oxidase inhibitor. More specifically, the present invention relates to a method for preparing a compound of the following Formula 2 by using an ester hydrolysis reaction and a recrystallization method: 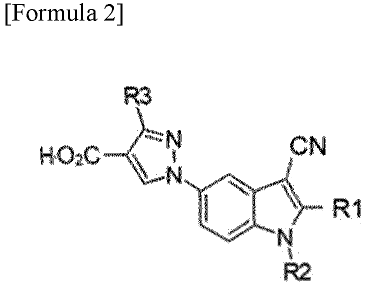 wherein,
R1 is hydrogen, halogen, C₁-C₇ alkyl, C₁-C₇ alkoxy-C₁-C₇ alkyl or phenyl;
R2 is hydrogen; C₁-C₇ alkyl unsubstituted or substituted by one or more substituents selected from halogen, C₃-C₇ cycloalkyl and O-R6; C₃-C₇ cycloalkyl; or 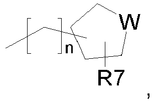 wherein R6 is C₁-C₄ alkyl, W is O or S, R7 is hydrogen or C₁-C₄ alkyl, and n is an integer of 0 to 3; and
R3 is hydrogen, halogen or C₁-C₇ alkyl.

### BACKGROUND ART

Xanthine oxidase is known as an enzyme which converts hypoxanthine to xanthine and further converts thus-formed xanthine to uric acid. Although most mammals have uricase, humans and chimpanzees do not, thereby uric acid is known to be the final product of purine metabolism (S. P. Bruce, Ann. Pharm., 2006, 40, 2187-2194). Sustained elevation of blood concentration of uric acid causes various diseases, representatively including gout.

As described above, gout is caused by an elevated level of uric acid in the body, indicating the condition in which uric acid crystals accumulated in cartilage, ligament and surrounding tissue induce severe inflammation and pain. Gout is a kind of inflammatory articular disease, and its incidence rate has steadily increased during past 40 years (N. L. Edwards, Arthritis & Rheumatism, 2008, 58, 2587-2590).

Accordingly, various studies have been conducted to develop new xanthine oxidase inhibitors, and Korean Patent Application Publication No. 10-2011-0037883 discloses a novel compound of the following formula, which is effective as a xanthine oxidase inhibitor:

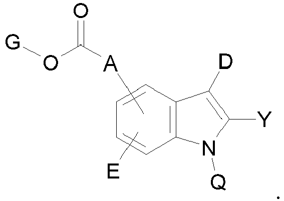

In the conventional process step of preparing an ester hydrolysate having xanthine oxidase inhibitory activity, excessive NaOH was used during the reaction. As a result, cyano group at the C3 position of the indole was hydrolyzed, and there was a problem in that an excessive amount of amide impurity was generated. In the crystallization process, it was not easy to remove the generated impurities, and excessive fume is generated due to the use of concentrated HCl, and there is a problem in that it is difficult to satisfy the temperature control acceptance criteria due to strong heat generation. In addition, the purification process has a problem in that the purification was not very effective in removing impurities, and it was not easy to remove THF solvent remaining in the drying process up to 720 ppm-which is the acceptance standard of the ICH guideline, and it was not suitable to manage the particle characteristics. Accordingly, it is necessary to develop an ester hydrolysis process that dramatically reduces impurities and an effective purification method.

### [Prior Document]

Patent Document: Korean Patent Application Publication No. 10-2011-0037883

### DISCLOSURE OF INVENTION

### TECHNICAL PROBLEM

Accordingly, the technical problem of the present invention is the provision of a novel method for preparing a compound of the following Formula 2 which is an excellent xanthine oxidase inhibitor in which residual impurities are remarkably reduced, THF solvent is removed below the allowable standard of the ICH guideline, and easy particle properties for formulation can be secured at the same time: 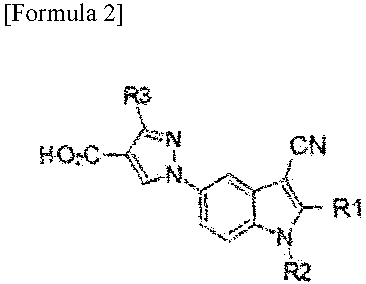 wherein R1, R2 and R3 are the same as defined herein.

### SOLUTION TO PROBLEM

To solve the above technical problem, there is provided a method for preparing a compound of the following Formula 2, comprising:
a first step of hydrolyzing a compound of Formula 1 by using NaOH dissolved in a mixed solvent of a protic solvent and tetrahydrofuran (THF) to obtain a compound of Formula 2; and
a second step of crystallizing the compound of Formula 2 obtained in the first step by using a mixed solvent of acetone and ethyl acetate: 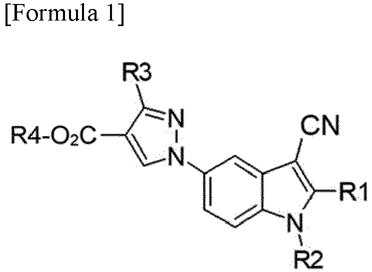 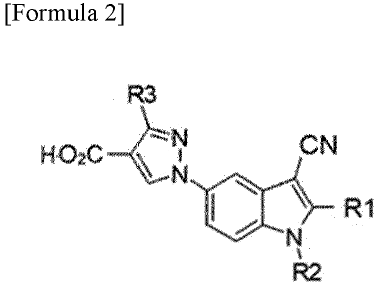
wherein,
   R1 is hydrogen, halogen, C₁-C₇ alkyl, C₁-C₇ alkoxy-C₁-C₇ alkyl or phenyl;
   R2 is hydrogen; C₁-C₇ alkyl unsubstituted or substituted by one or more substituents selected from halogen, C₃-C₇ cycloalkyl and O-R6; C₃-C₇ cycloalkyl; or 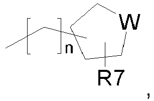 wherein R6 is C₁-C₄ alkyl, W is O or S, R7 is hydrogen or C₁-C₄ alkyl, and n is an integer of 0 to 3;
   R3 is hydrogen, halogen or C₁-C₇ alkyl; and
   R4 is C₁-C₇ alkyl or C₃-C₇ cycloalkyl.

According to one embodiment of the present invention, R2 may be unsubstituted C₁-C₇ alkyl, but is not limited thereto.

According to one embodiment of the present invention, R2 may be isopropyl, but is not limited thereto.

According to one embodiment of the present invention, the protic solvent may be one or more selected from the group consisting of methanol, ethanol, propanol, butanol and acetic acid, but is not limited thereto.

According to one embodiment of the present invention, the protic solvent may be methanol, but is not limited thereto.

According to one embodiment of the present invention, the NaOH of the first step may be used at a concentration of 8 N to 12 N, and most preferably about 10 N, but is not limited thereto.

According to one embodiment of the present invention, the first step may further comprise a step of crystallization using HCl and ethyl acetate, but is not limited thereto.

According to one embodiment of the present invention, the HCl may be used at a concentration of 2 N to 12 N, and more preferably about 6 N, but is not limited thereto.

According to one embodiment of the present invention, the second step may further comprise a step of adding NaOH dropwise, filtrating and adding HCl dropwise to a filtrate, but is not limited thereto.

According to one embodiment of the present invention, the HCl may be added dropwise at a temperature of 48°C to 62°C, and more preferably 48°C to 52°C, but is not limited thereto.

According to one embodiment of the present invention, the HCl may be added dropwise for 3 hours to 5 hours, and more preferably about 4 hours, but is not limited thereto.

### EFFECTS OF THE INVENTION

The preparation method of the present invention can remarkably reduce residual impurity and remarkably reduce the amount of residual solvent compared to the conventional method in the preparation of the compound of Formula 2.

### MODE FOR THE INVENTION

Hereinafter, the present invention is explained in more detail with the following examples. However, it must be understood that the protection scope of the present invention is not limited to the examples.

### Example 1: Results of 1-(3-cyano-1-isopropyl-indol-5-yl)pyrazole-4-carboxylic acid reaction test

### Example 1.1 Hydrolysis experiment in acidic condition

In the case of a reaction in which the ester is hydrolyzed in 1-(3-cyano-1-isopropyl-indol-5-yl)pyrazole-4-carboxylic acid ethyl ester, it has been known that hydrolysis occurs not only under basic conditions but also under acidic conditions. Therefore, the following experiments were conducted to confirm the reactivity and reaction selectivity under acidic conditions (Table 1). For the experiments, 5 g of 1-(3-cyano-1-isopropyl-indol-5-yl)pyrazole-4-carboxylic acid ethyl ester, AlCl₃ which is a Lewis acid and HCl which is a Bronsted-Lowry acid were used.

**[Table 1] Acid hydrolysis reaction conditions and HPLC results**

| Solvent & reagent | | | | Reaction condition | | HPLC Peak Area % | | | |
|---|---|---|---|---|---|---|---|---|---|
| ACN | Water | NaI | AlCl₃ | Temp | Time | | | | |
| 5.5 fold | 0.5 fold | 1.5 eq. | 1.5 eq. | reflux | 2 h | No reaction | | | |
| | | | | | 20 h | | | | |

| Solvent & reagent | | | | Reaction condition | | HPLC Peak Area % | | | |
|---|---|---|---|---|---|---|---|---|---|
| 4 N HCl in water | | 4 N HCl in dioxane | | Temp | Time | GD-IMP-1 | GD67 | UK | GD65 |
| 3 fold | | 3 fold | | reflux | 2 h | 23.8 | 40.7 | 10.0 | 25.5 |

| | | | | | | | | | |
|---|---|---|---|---|---|---|---|---|---|
| GD-IMP-1: 1-(3-Carbamoyl-1-isopropyl-1H-indol-5-yl)-1H-pyrazole-4-carboxylic acid UK: Unknown impurity GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester | | | | | | | | | |

In the case of the reaction using AlCl₃, the reaction did not proceed at all even after 20 hours. In the case ofHCl, after 2 hours of the reaction, it was confirmed by IPC (in process control) that 25.5% of 1-(3-cyano-1-isopropyl-indol-5-yl)pyrazole-4-carboxylic acid ethyl ester remained. As a result, the reactivity was lower than that of the existing reaction conditions. In addition, when the production amount of 1-(3-carbamoyl-1-isopropyl-1H-indol-5-yl)-1H-pyrazole-4-carboxylic acid was 23.8%, that of 1-(3-cyano-1-isopropyl-indol-5-yl)pyrazole-4-carboxylic acid was 40.7%. As such, it was judged that the reaction selectivity is also low. Last, the problem of generating unknown impurity, which was not observed in the existing reaction, was also found. Therefore, it was determined that the basic condition is more preferable than the acidic condition.

### Example 1.2. NaOH equivalent control experiment

In the existing process, 5 equivalents were used in the reaction of 1-(3-cyano-1-isopropyl-indol-5-yl)pyrazole-4-carboxylic acid. It was thought that excessive use of NaOH would contribute to the formation of 1-(3-carbamoyl-1-isopropyl-1H-indol-5-yl)-1H-pyrazole-4-carboxylic acid. Therefore, the experiment using 1-(3-cyano-1-isopropyl-indol-5-yl)pyrazole-4-carboxylic acid ethyl ester (5 g) to check whether the reaction is complete even if the number of equivalents is reduced was carried out (Table 2).

**[Table 2] Conditions and results of 1-(3-cyano-1-isopropyl-indol-5-yl)pyrazole-4-carboxylic acid reaction**

| Run | THF (fold) | MeOH (fold) | Base (equiv) | Reaction time | HPLC Peak Area % | | | | | THF (%) | Yield (%) |
|---|---|---|---|---|---|---|---|---|---|---|---|
| | | | | | GD-IMP-1 | GD67 | GD-IMP-2 | GD-IMP-3 | GD65 | | |
| Example 1-2-1 | 1.56 | 1.56 | 2 | 1 h | 0.02 | 60.96 | 35.87 | 0.22 | 2.94 | 4.11 | 96.9 |
| | | | | 2 h | 0.40 | 89.23 | 8.43 | 0.21 | 0.63 | | |
| | | | | 3 h | 0.47 | 98.96 | ND | 0.19 | 0.08 | | |
| | | | | F.C. | 0.80 | 99.02 | ND | 0.18 | ND | | |
| Example 1-2-2 | 1.56 | 1.56 | 3 | 1 h | 0.31 | 67.13 | 30.18 | 0.21 | 2.17 | 3.63 | 96.3 |
| | | | | 2 h | 0.48 | 98.65 | 0.61 | 0.21 | 0.06 | | |
| | | | | 3 h | 0.79 | 99.01 | ND | 0.20 | ND | | |
| | | | | F.C. | 1.14 | 98.65 | ND | 0.21 | ND | | |
| Example 1-2-3 | 1.56 | 1.56 | 5 | 1 h | 0.33 | 67.94 | 28.74 | 0.24 | 2.76 | 2.97 | 93.9 |
| | | | | 2 h | 0.68 | 99.00 | 0.08 | 0.23 | 0.01 | | |
| | | | | 3 h | 1.17 | 98.63 | ND | 0.20 | ND | | |
| | | | | F.C. | 2.31 | 97.5 | ND | 0.21 | ND | | |

| | | | | | | | | | | | |
|---|---|---|---|---|---|---|---|---|---|---|---|
| GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester GD-IMP-1: 1-(3-Carbamoyl-1-isopropyl-1H-indol-5-yl)-1H-pyrazole-4-carboxylic acid GD-IMP-2: Methyl-1-(3-cyano-1-isopropyl-1H-indol-5-yl)-1H-pyrazole-4-carboxylate GD-IMP-3: 1-(3,3'-Dicyano-1'-isopropyl-1'H-[1,5'-biindol]-5-yl)-1H-pyrazole-4-carboxylic acid F.C.: Filtercake | | | | | | | | | | | |

Conditions in which only the number of equivalents of NaOH were reduced little by little were applied (Examples 1-2-1 and 1-2-2), and IPC was performed at 1-hour intervals to confirm conversion. As the number of NaOH equivalents increases, the reaction proceeds relatively quickly, but the impurity (1-(3-carbamoyl-1-isopropyl-1H-indol-5-yl)-1H-pyrazole-4-carboxylic acid) tends to increase, and it was determined that the impurity (1-(3,3'-dicyano-1'-isopropyl-1'H-[1,5'-biindol]-5-yl)-1H-pyrazole-4-carboxylic acid) is irrelevant to the equivalence of NaOH.

### Example 1.3. Co-base application experiment

To reduce the generation of the impurity (1-(3-carbamoyl-1-isopropyl-1H-indol-5-yl)-1H-pyrazole-4-carboxylic acid) at the time of 1-(3-cyano-1-isopropyl-indol-5-yl)pyrazole-4-carboxylic acid reaction according to the use of strong base such as NaOH, the experiments for checking the change in reaction selectivity-in which the amount of NaOH was reduced and the mild base was treated together while maintaining the total number of equivalents of the base-were conducted. 2 Types of inorganic bases and 4 types of organic bases (2 types of aliphatic amines/2 types of heterocyclic amines) were selected, and the experiments were carried out on 3 g scale of 1-(3-cyano-1-isopropyl-indol-5-yl)pyrazole-4-carboxylic acid ethyl ester (Table 3).

**[Table 3] Experimental conditions and results of 1-(3-cyano-1-isopropyl-indol-5-yl)pyrazole-4-carboxylic acid co-base screening**

| Run | THF (fold) | MeO H (fold) | Base (1:1) (5 equiv) | | Reaction time | HPLC Peak Area % | | | | | Comment |
|---|---|---|---|---|---|---|---|---|---|---|---|
| | | | | | | GD-IMP-1 | GD67 | GD-IMP-2 | GD-IMP-3 | GD65 | |
| Example 1-3-1 | 1.56 | 1.56 | NaOH (5 eq.) | | 3 h | 1.96 | 97.85 | ND | 0.18 | 0.02 | 3 g scale (carousel) |
| | | | | | 6 h | 3.10 | 96.38 | ND | 0.17 | 0.03 | |
| Example 1-3-2 | 1.56 | 1.56 | NaOH | K₃PO₄ | 3 h | 0.55 | 99.24 | ND | 0.20 | ND | |
| | | | | | 6 h | 0.83 | 98.97 | ND | 0.20 | ND | |
| Example 1-3-3 | 1.56 | 1.56 | NaOH | NaHCO₃ | 3 h | 0.48 | 99.32 | ND | 0.20 | ND | |
| | | | | | 6 h | 0.80 | 98.96 | ND | 0.20 | ND | |
| Example 1-3-4 | 1.56 | 1.56 | NaOH | TEA | 3 h | 0.43 | 99.27 | 0.10 | 0.20 | 0.02 | |
| | | | | | 6 h | 0.72 | 99.04 | ND | 0.20 | ND | |
| Example 1-3-5 | 1.56 | 1.56 | NaOH | DIPEA | 3 h | 0.43 | 98.97 | 0.38 | 0.20 | ND | |
| | | | | | 6 h | 0.71 | 99.04 | ND | 0.20 | ND | |
| Example 1-3-6 | 1.56 | 1.56 | NaOH | Pyridine | 3 h | 0.47 | 99.347 | ND | 0.20 | ND | |
| | | | | | 6 h | 0.76 | 99.05 | ND | 0.20 | ND | |
| Example 1-3-7 | 1.56 | 1.56 | NaOH | DMAP | 3 h | 0.42 | 99.31 | 0.07 | 0.20 | ND | |
| | | | | | 6 h | 0.70 | 99.10 | ND | 0.20 | ND | |

| | | | | | | | | | | | |
|---|---|---|---|---|---|---|---|---|---|---|---|
| GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester GD-IMP-1: 1-(3-Carbamoyl-1-isopropyl-1H-indol-5-yl)-1H-pyrazole-4-carboxylic acid GD-IMP-2: Methyl-1-(3-cyano-1-isopropyl-1H-indol-5 -yl)-1H-pyrazole-4-carboxylate GD-IMP-3: 1-(3,3'-Dicyano-1'-isopropyl-1'H-[1,5'-biindol]-5-yl)-1H-pyrazole-4-carboxylic acid | | | | | | | | | | | |

In the case of K₃PO₄ having a large molecular weight, the input weight was 4.94 g based on 3 g of 1-(3-cyano-1-isopropyl-indol-5-yl)pyrazole-4-carboxylic acid ethyl ester, which is a larger amount than the reactant. This large amount caused a problem in that stirring was not easy (Example 1-3-2). In addition, as the reaction progressed, it was observed that the viscosity of the reaction mixture increased, and this resulted in poor stirring, and slightly delayed conversion of methyl ester (Examples 1-3-4 and 1-3-5). Overall, there was no significant change in the generation of the impurity (1-(3-carbamoyl-1-isopropyl-1H-indol-5-yl)-1H-pyrazole-4-carboxylic acid), so it was determined that there is little need for co-base treatment (Examples 1-3-2 to 1-3-7).

### Example 1.4. Solvent screening experiments

**[Table 4] Experimental conditions and results of 1-(3-cyano-1-isopropyl-indol-5-yl)pyrazole-4-carboxylic acid solvent screening (1-(3-cyano-1-isopropyl-indol-5-yl) pyrazole-4-carboxylic acid ethyl ester 5 g scale)**

| Run | Solvent & reagent | | | | Reaction condition | | HPLC Peak Area % | | | | | | THF content (%) | NMR Assay (%) | Net Yield (%) |
|---|---|---|---|---|---|---|---|---|---|---|---|---|---|---|---|
| | THF | EtOH | ACN | 10 N NaOH | Temp (°C) | Time | GD-IMP-1 | | GD67 | | GD-IMP-2 | GD65 | | | |
| Ref. | THF: 3 fold | | | 2 eq | 20 | 3 h | 0.17 | | 92.91 | | 6.62 | 0.18 | 2.59 | 93.8 | 86.8 |
| | | | | | | 5 h | 0.23 | | 97.52 | | 2.19 | 0.05 | | | |
| | MeOH: 3 fold | | | | | 23 h | 0.81 | | 97.40 | | 0.20 | ND | | | |
| | | | | | | f.c. | 0.39 | | 99.35 | | 0.24 | 0.03 | | | |
| Example 1-4-1 | 6 fold | - | - | 2 eq | 20 | No reaction | | | | | | | | | |
| Example 1-4-2 | 3 fold | - | 3 fold | 2 eq | 20 | | | | | | | | | | |
| Example 1-4-3 | 3 fold | 3 fold | - | 2 eq | 20 | 1 h | 0.18 | 74.85 | | 18.8 | | 3.23 | 2.57 | 90.1 | 79.0 |
| | | | | | | 3 h | 0.66 | 96.68 | | 1.72 | | 0.49 | | | |
| | | | | | | 5 h | 1.16 | 97.93 | | 0.27 | | 0.11 | | | |
| | | | | | | 23 h | 4.16 | 95.20 | | 0.18 | | ND | | | |

| | | | | | | | | | | | | | | | |
|---|---|---|---|---|---|---|---|---|---|---|---|---|---|---|---|
| GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester GD-IMP-1: 1-(3-Cyano-1-isopropyl-indol-5-yl)pyrazole-4-carboxylic acid GD-IMP-2: Methyl-1-(3-cyano-1-isopropyl-1H-indol-5-yl)-1H-pyrazole-4-carboxylate | | | | | | | | | | | | | | | |

From the above results, when only the polar aprotic solvent was used, the reaction did not proceed at all (Examples 1-4-1 and 1-4-2). When EtOH is used instead of MeOH, an unknown impurity which has the retention time similar to that of the impurity (methyl-1-(3-cyano-1-isopropyl-1H-indol-5-yl)-1H-pyrazole-4-carboxylate) was generated, and it was estimated as a reaction intermediate by considering the tendency to rapidly decrease after formation at the initial stage of the reaction (Example 1-4-3). When comparing to the result of the Ref. in Table 4, 1-(3-cyano-1-isopropyl-indol-5-yl)pyrazole-4-carboxylic acid generated at the same time was increased compared to the use of MeOH solvent. In addition, it was observed that the filterability deteriorated compared to the existing conditions during the filtration process, but the filtercake color was improved (pale brown→off white). It was confirmed that the yield and NMR assay values measured after drying were lowered. From the above results, it was confirmed that a protic solvent was essential for the progress of the reaction, and the application of THF and MeOH co-solvent system showed the best results.

### Example 2: Results of 1-(3-cyano-1-isopropyl-indol-5-yl)pyrazole-4-carboxylic acid) crystallization test

### Example 2.1. Selection of co-solvent of 1-(3-cyano-1-isopropyl-indol-5-yl)pyrazole-4-carboxylic acid crystallization

In order to remove 1-(3-cyano-1-isopropyl-indol-5-yl)pyrazole-4-carboxylic acid from the crystallization process, experiments were conducted to select a co-solvent to be used together during the crystallization process (Table 5).

**[Table 5] Screening experiment for co-solvent of 1-(3-cyano-1-isopropyl-indol-5-yl)pyrazole-4-carboxylic acid crystallization process**

| Solvent | HPLC Peak Area % | | | |
|---|---|---|---|---|
| | GD-IMP-1 | GD67 | GD-IMP-2 | GD-IMP-3 |
| Initial | 0.638 | 99.053 | 0.309 | N/D |
| Toluene | 0.283 | 99.198 | 0.254 | N/D |
| EtOAc | 0.105 | 99.600 | 0.145 | N/D |
| MTBE | 0.155 | 99.626 | 0.188 | N/D |
| DCM | 0.389 | 99.276 | 0.260 | N/D |
| Heptane | 0.303 | 99.382 | 0.255 | N/D |

| | | | | |
|---|---|---|---|---|
| GD67: 1-(3-Cyano-1-isopropyl-indol-5-yl)pyrazole-4-carboxylic acid GD-IMP-1: 1-(3-Carbamoyl-1-isopropyl-1H-indol-5-yl)-1H-pyrazole-4-carboxylic acid GD-IMP-2: Methyl-1-(3-cyano-1-isopropyl-1H-indol-5-yl)-1H-pyrazole-4-carboxylate GD-IMP-3: 1-(3,3'-Dicyano-1'-isopropyl-1'H-[1,5'-biindol]-5-yl)-1H-pyrazole-4-carboxylic acid | | | | |

The initial content value of the 1-(3-cyano-1-isopropyl-indol-5-yl)pyrazole-4-carboxylic acid sample was 0.638%, and it was confirmed that the initial content value was decreased to 0.105% when ethyl acetate was used (Table 5). In addition, it was confirmed that the impurity (methyl-1-(3-cyano-1-isopropyl-1H-indol-5-yl)-1H-pyrazole-4-carboxylate) also decreased from 0.309% to 0.145%. Furthermore, DCM, heptane and toluene also decreased 1-(3-cyano-1-isopropyl-indol-5-yl)pyrazole-4-carboxylic acid, but showed lower reduction ratios (%) compared to ethyl acetate. As a result, ethyl acetate was selected as a co-solvent to be used in the crystallization process of 3-cyano-1-isopropyl-indol-5-yl)pyrazole-4-carboxylic acid.

### Example 2.1.2. Solvent screening for 1-(3-Cyano-1-isopropyl-indol-5-yl)pyrazole-4-carboxylic acid crystallization

After reaction of 1-(3-cyano-1-isopropyl-indol-5-yl)pyrazole-4-carboxylic acid, conc. HCl was used. Using a high concentration of HCl was judged to be undesirable in terms of work safety. In addition, in the experimental aspect, it was difficult to control the pH, and a large amount of generated fume made it difficult to secure the field of view. To improve this, it was replaced with 3 N HCl, and an appropriate amount of an organic solvent was added to enhance the purification effect. In order to ascertain the difference by the crystallization method, all reactions were carried out under the same conditions and scale as much as possible. In order to clearly ascertain the difference before and after crystallization, HPLC analysis of the reaction solution was performed immediately before crystallization, and this was compared with filtercake (Table 6).

**[Table 6] Reaction conditions, crystallization method and solvent types of 1-(3-cyano-1-isopropyl-indol-5-yl)pyrazole-4-carboxylic acid**

| Run | Solvent & reagent | | | Reaction condition | | HPLC Peak Area % | | | | | THF content (%) | Solvent content (%) | Gross Yield (%) |
|---|---|---|---|---|---|---|---|---|---|---|---|---|---|
| | THF | MeOH | 10 N NaOH | Temp. (°C) | Time (h) | GD-IMP-1 | GD67 | GD-IMP-2 | GD-IMP-3 | GD65 | | | |
| Example 2-1-1 | 3 fold | 3 fold | 2.5 eq. | 30 | 3 | 0.44 | 99.19 | 0.13 | 0.19 | 0.05 | 0.64 | 0.33 (ACN) | 93.6 |
| | | | | | f.c. | 0.15 | 99.57 | 0.09 | 0.20 | ND | | | |
| Example | 3 fold | 3 fold | 2.5 eq. | 30 | 3 | 0.46 | 99.27 | 0.07 | 020 | ND | 0.61 | 0.33 | 88.1 |
| 2-1-2 | | | | | f.c. | 0.17 | 99.59 | 0.06 | 0.19 | ND | | (acetone) | |
| Example 2-1-3 | 3 fold | 3 fold | 2.5 eq. | 30 | 3 | 0.50 | 99.25 | 0.05 | 0.20 | ND | 0.88 | 0.15 (EtOAc) | 87.6 |
| | | | | | f.c. | 0.17 | 99.64 | ND | 0.19 | ND | | | |
| Example 2-1-4 | 3 fold | 3 fold | 2.5 eq. | 30 | 3 | 0.51 | 99.27 | 0.03 | 0.20 | ND | 0.82 | 0.10 (EtOAc) | 87.2 |
| | | | | | f.c. | 0.18 | 99.63 | 0.02 | 0.18 | ND | | | |
| Example 2-1-5 | 3 fold | 3 fold | 2.5 eq. | 30 | 3 | 0.49 | 99.28 | 0.04 | 0.19 | ND | 1.03 | 0.36 (MeOH) | 90.5 |
| | | | | | f.c. | 0.16 | 99.64 | 0.03 | 0.16 | ND | | | |
| Example 2-1-6 | 3 fold | 3 fold | 2.5 eq. | 30 | 3 | 0.46 | 99.26 | 0.09 | 0.19 | ND | 0.67 | 0.37 (ACN) | 86.6 |
| | | | | | f.c. | 0.13 | 99.65 | 0.07 | 0.15 | ND | | | |

| | | | | | | | | | | | | | |
|---|---|---|---|---|---|---|---|---|---|---|---|---|---|
| Example 2-1-1: ACN (1.5 fold), 3N HCl input and then keeping (30°C, pH = 1.8, 30 min)→Low temperature keeping (5°C, 30 min) Example 2-1-2: Acetone (1.5 fold), 3N HCl input and then keeping (30°C, pH = 1.6, 30 min)→Low temperature keeping (5°C, 30 min) Example 2-1-3: H2O (1 fold), EA (1.5 fold), 3N HCl input and then keeping (30°C, pH = 1.6, 30 min) → room temperature keeping (25°C, 2 h) Example 2-1-4: EA (1.5 fold), 3N HCl input and then keeping (30°C, pH = 1.6, 30 min)→Low temperature keeping (0°C, 2 h) Example 2-1-5: MeOH (1.5 fold), 3N HCl mixed solution added and then keeping (30°C, pH = 1.6, 30 min) → room temperature keeping (23°C, 30 min) Example 2-1-6: ACN (1.5 fold), 3N HCl mixed solution added and keeping (30°C, pH = 1.6, 30 min) → room temperature keeping (23°C, 30 min) GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester GD-IMP-1: 1-(3-Carbamoyl-1-isopropyl-1H-indol-5-yl)-1H-pyrazole-4-carboxylic acid GD-IMP-2: Methyl-1-(3-cyano-1-isopropyl-1H-indol-5 -yl)-1H-pyrazole-4-carboxylate GD-IMP-3: 1-(3,3'-dicyano-1'-isopropyl-1'H-[1,5'-biindol]-5-yl)-1H-pyrazole-4-carboxylic acid | | | | | | | | | | | | | |

As the above results, the yield change according to the aging temperature and aging time was confirmed (Examples 2-1-1 to 2-1-6). The solubility of 1-(3-cyano-1-isopropyl-indol-5-yl)pyrazole-4-carboxylic acid in the organic solvents used at this time was 0.0001 g/ml or less for ACN, MeOH and EtOAc, and 0.0001-0.001 g/ml for acetone. Therefore, the comparative experiments were conducted by assuming that the effect on yield was insignificant depending on the type of solvent. When comparing the purification effect according to the solvent type under the same reaction temperature and aging conditions, only the purification effect on 1-(3-cyano-1-isopropyl-indol-5-yl)pyrazole-4-carboxylic acid was observed, and the degree of purification was similar to about 65%, but EtOAc showed the best results in terms of residual solvent (Examples 2-1-1, 2-1-2 and 2-1-4). In order to minimize the increase of 1-(3-cyano-1-isopropyl-indol-5-yl)pyrazole-4-carboxylic acid, it is necessary to lower the pH as quickly as possible after confirming the completion point of the reaction by IPC. As such, in order to advance the time of aqueous HCl input, the experiments in which a mixture of an organic solvent and 3 N HCl was added dropwise after completion of the reaction were carried out (Examples 2-1-5 and 2-1-6). However, there was no significant difference between the case of adding ACN and 3 N HCl sequentially and the case of adding a mixed solution of ACN and 3 N HCl (Examples 2-1-1 and 2-1-6).

### Example 3: Preparation of 1-(3-cyano-1-isopropyl-indol-5-yl)pyrazole-4-carboxylic acid

THF (554 kg), 1-(3-cyano-1-isopropyl-indol-5-yl)pyrazole-4-carboxylic acid ethyl ester (312 kg) and MeOH (624 L) were added to a reactor, and 10 NNaOH (386 kg) was slowly added thereto. Since it is an exothermic reaction, the reactants were added for about 1 hour, taking care not to exceed 27°C of the internal temperature. After completion of the dropwise addition, the reaction was carried out in the range of 21-27°C, and IPC was performed. After completion of the reaction, purified water (624 L) and EtOAc (197 kg) were added, and then 3 N HCl (1,048 kg) was slowly added dropwise while maintaining 30-35°C. In the above process, 3 N HCl was added dropwise by dividing the first and second phases, and the first dropwise addition was carried out until the pH = 5-6 for the 1st nucleation of the solid. After stirring the reaction mixture in which a solid was produced for 30 minutes, the second dropwise addition was performed until the pH = 2-3 to proceed with particle size growth. After completion of the dropwise addition, the obtained product was cooled to room temperature, kept at this temperature for 30 minutes and filtered. The filtered solid was washed with purified water (624 L) and dried under nitrogen and vacuum to obtain 1-(3-cyano-1-isopropyl-indol-5-yl)pyrazole-4-carboxylic acid (273.2 kg, 95.9% gross yield).

### Example 4: Recrystallization of 1-(3-cyano-1-isopropyl-indol-5-yl)pyrazole-4-carboxylic acid

### Example 4.1. Solvent screening for 1-(3-cyano-1-isopropyl-indol-5-yl)pyrazole-4-carboxylic acid

In view of the result that 1-(3-cyano-1-isopropyl-indol-5-yl)pyrazole-4-carboxylic acid was effectively removed when EtOAc was used in the crystallization process of 1-(3-cyano-1-isopropyl-indol 5-yl)pyrazole-4-carboxylic acid, the same was intended to be used in the recrystallization of 1-(3-cyano-1-isopropyl-indol-5-yl)pyrazole-4-carboxylic acid. After recrystallization was performed by using acetate-type solvents similar to EtOAc, the experiments were performed to determine the optimal solvent by analyzing the obtained filtercake (Table 7).

**[Table 7] Solvent screening for 1-(3-cyano-1-isopropyl-indol-5-yl)pyrazole-4-carboxylic acid process**

| Run | Solvent | | HPLC Peak Area % | | | | | Acetate content (%) | Acetone content (%) | Gross Yield (%) |
|---|---|---|---|---|---|---|---|---|---|---|
| | | Filterability | GD-IMP-1 | GD67 | GD-IMP-2 | GD-IMP-3 | GD65 | | | |
| Example 4-1-1 | n-Butyl acetate | Very bad | 0.91 | 98.62 | 0.02 | 0.44 | 0.01 | 2.01 | 0.91 | 78.4% |
| Example 4-1-2 | Isobutyl acetate | Very bad | 1.39 | 98.06 | 0.02 | 0.54 | ND | 5.59 | 0.65 | 92.9% |
| Example 4-1-3 | Methyl acetate | Good | 0.42 | 99.00 | 0.03 | 0.54 | 0.01 | 3.13 | 0.07 | 70.8% |
| Example 4-1-4 | Ethyl acetate | Medium | 0.65 | 98.73 | 0.02 | 0.59 | ND | 5.52 | 0.16 | 74.6% |
| Example 4-1-5 | n-Propyl acetate | Bad | 0.69 | 98.70 | 0.02 | 0.55 | 0.03 | 3.74 | 0.59 | 75.0% |
| Example 4-1-6* | Methyl acetate | Good | 0.26 | 99.17 | 0.04 | 0.54 | ND | 2.81 | 0.43 | 86.5% |
| Example 4-1-7* | Ethyl acetate | Good | 0.26 | 99.15 | 0.03 | 0.57 | ND | 4.13 | 0.33 | 88.0% |

| | | | | | | | | | | |
|---|---|---|---|---|---|---|---|---|---|---|
| * Scale up the existing 9 g scale experiment to 22.5 g scale and re-experiment, Use of 250 ml glass type reactor GD67: 1-(3-Cyano-1-isopropyl-indol-5-yl)pyrazole-4-carboxylic acid GD65: 1-(3-Cyano-1-isopropyl-indol-5-yl)pyrazole-4-carboxylic acid ethyl ester GD-IMP-1: 1-(3-Carbamoyl-1-isopropyl-1H-indol-5-yl)-1H-pyrazole-4-carboxylic acid GD-IMP-2: Methyl-1-(3-cyano-1-isopropyl-1H-indol-5-yl)-1H-pyrazole-4-carboxylate GD-IMP-3: 1-(3,3'-Dicyano-1'-isopropyl-1'H-[1,5'-biindol]-5-yl)-1H-pyrazole-4-carboxylic acid | | | | | | | | | | |

As a result of the experiments of 1-(3-cyano-1-isopropyl-indol-5-yl)pyrazole-4-carboxylic acid on 9 g scale, the following tendency was confirmed: when solvents having many carbon atoms substituted at the ester position were used, the viscosity of the reaction mixture was increased, and the filterability tendency was decreased, thereby decreasing the purification effect (Examples 4-1-1 to 4-1-5). By the use of methyl acetate and ethyl acetate which showed excellent results in the previous experiment, the re-experiments for scale-up and verification were performed (Examples 4-1-6 and 4-1-7). There may be differences from the previous results due to differences in the reactor type and scale, but when only the two scale-up experiments were compared, both methyl acetate and ethyl acetate solvents had good filtration tendencies. Similar results were obtained in terms of impurity and purity, and in the case of residual content after drying, the content of acetone used as a co-solvent was 4.13% in the experiment using ethyl acetate and 2.81% in the experiment using methyl acetate. In terms of yield, in the experiment using ethyl acetate, the yield was 88.0%, which was 1.5% higher than 86.5% of methyl acetate. Ethyl acetate is the solvent used in the preparation of 1-(3-cyano-1-isopropyl-indol-5-yl)pyrazole-4-carboxylic acid ethyl ester and 1-(3-cyano-1-isopropyl-indol-5-yl)pyrazole-4-carboxylic acid, and is used universally. As such, ethyl acetate was selected as a co-solvent of acetone in the crystallization solvent condition of 1-(3-cyano-1-isopropyl-indol-5-yl)pyrazole-4-carboxylic acid.

### Example 4.2. Condition search for effective removal of residual solvent (I) - Temperature and time of dropwise adding

At the time of recrystallization of 1-(3-cyano-1-isopropyl-indol-5-yl)pyrazole-4-carboxylic acid, as represented in Table 8, 1 N NaOH solution was used for the complete dissolution of 1-(3-cyano-1-isopropyl-indol-5-yl)pyrazole-4-carboxylic acid, EtOAc was used for the removal of 1-(3-carbamoyl-1-isopropyl-1H-indol-5-yl)-1H-pyrazole-4-carboxylic acid, and acetone was used for the homogeneous mixture. The recrystallization was carried out in a co-solvent system of acetone, water and EtOAc.

As a result, THF-which was contained in a large amount in the existing method-was effectively removed, but there was a problem in which acetone remained. As such, conditions for effectively reducing acetone were searched. All experiments were performed in a 500 ml glass type reactor using 40 g of 1-(3-cyano-1-isopropyl-indol-5-yl)pyrazole-4-carboxylic acid ethyl ester (Table 8). The dryer used a glass filter, and as for the drying method nitrogen pressure was applied. After confirming that there was no further change in weight by checking the weight of the cake, drying was terminated.

**[Table 8] Experimental conditions and results of 1-(3-cyano-1-isopropyl-indol-5-yl)pyrazole-4-carboxylic acid process**

| Run | Solvent (ml) | | | | Solution added dropwise (ml) | | Temp. (°C) | Time (min) | Acetone content (%) |
|---|---|---|---|---|---|---|---|---|---|
| | Acetone | Water | EA | 1N NaOH | 6N HCl | Acetone | | | |
| Example 4-2-1 | 120 | 0 | 13.2 | 133 | 24 | 0 | 25 | 30 | 2.03 |
| Example 4-2-2 | | | | | | | 50 | 30 | 0.94 |
| Example 4-2-3 | | | | | | | | 90 | 0.78 |
| Example 4-2-4 | | | | | | | | 180 | 0.52 |
| Example 4-2-5 | | | | | | | 60 | 30 | 0.56 |
| Example 4-2-6 | | | | | | | | 90 | 0.43 |
| Example 4-2-7 | 60 | 60 | 13.2 | 133 | 24 | 0 | 25 | 30 | 1.39 |
| Example 4-2-8 | | | | | | | 50 | 30 | 0.62 |
| Example 4-2-9 | | | | | | | 60 | 30 | 0.46 |

From the above results of the experiments, under the same conditions, as the temperature of adding 6 N HCl dropwise increased, the removal of acetone in API (active pharmaceutical ingredients, 1-(3-cyano-1-isopropyl-indol-5-yl)pyrazole-4-carboxylic acid) was effective (Examples 4-2-1, 4-2-2, 4-2-5, 4-2-7, 4-2-8 and 4-2-9). At this time, it was observed that the higher the temperature, the larger the generated particles, thereby improving the filterability. In addition, at the same temperature of dropwise addition, as the dropwise addition time increased, the acetone removal of 1-(3-cyano-1-isopropyl-indol-5-yl)pyrazole-4-carboxylic acid was more effective, and there was no significant difference in filterability in this process (Examples 4-2-1, 4-2-2, 4-2-3, 4-2-5 and 4-2-6). In terms of residual solvent and filterability, it was considered desirable to proceed with crystallization at high temperature and by lengthening aqueous HCl dropwise addition time.

### Example 4.4. Scale-up of 1-(3-cyano-1-isopropyl-indol-5-yl)pyrazole-4-carboxylic acid

As the final API preparing process, in the existing method, the crystalline particle size was very small (less than 50 µm), the shape was not uniform, and the particle size was not uniform. Specifically, in the final process, after adding 1-(3-cyano-1-isopropyl-indol-5-yl)pyrazole-4-carboxylic acid in a solid state, purified water was added thereto and stirred under reflux conditions. However, because the stirring was carried out in the undissolved state, there was a disadvantage in that the physical properties of the crystals could not be controlled. In addition, considering the contamination of foreign substances that may occur in the previous process step and ROI (residue on ignition), a micro filtration step was necessary before final crystallization. Therefore, in the preparation process of 1-(3-cyano-1-isopropyl-indol-5-yl)pyrazole-4-carboxylic acid according to the present invention, 1 N NaOH was added dropwise to a mixed solvent of acetone and ethyl acetate to completely dissolve 1-(3-cyano-1-isopropyl-indol-5-yl)pyrazole-4-carboxylic acid, after the micro filtration, and the process was carried out with 6 N HCl at the set crystallization temperature and pH to control the physical properties of the final API and to minimize contamination of foreign substances.

### Example 5: Preparation of 1-(3-cyano-1-isopropyl-indol-5-yl)pyrazole-4-carboxylic acid

After adding acetone (809 kg), 1-(3-cyano-1-isopropyl-indol-5-yl)pyrazole-4-carboxylic acid (344 kg) and EtOAc (102 kg), 1 N NaOH (1204 L) was slowly added dropwise over 1 hour while maintaining 10°C or less of the internal temperature and pH of 13.6 or less. After the addition was completed, the reaction mixture was stirred for an additional 30 minutes to dissolve as much as possible, and then microfiltered 6 N HCl (2 kg) was added dropwise to the microfiltered reaction mixture to lower the pH to 7.5-8, and then the temperature was raised. While maintaining the temperature of the reaction mixture at 48-52 °C, microfiltered 6 N HCl (197 kg) was slowly added dropwise over 4 hours to adjust pH = 4.5-5. After confirming the pH, cooling was started, the temperature of the reaction mixture dropped to room temperature, and then filtration was performed. The filtered solid was washed twice with purified water (688 L X 2) and dried under nitrogen and vacuum to obtain 1-(3-cyano-1-isopropyl-indol-5-yl)pyrazole-4-carboxylic acid (321.5 kg, 93.5% gross yield).

## Claims

1. A method for preparing a compound of the following Formula 2, comprising:
a first step of hydrolyzing a compound of Formula 1 by using NaOH dissolved in a mixed solvent of a protic solvent and tetrahydrofuran (THF) to obtain a compound of Formula 2; and
a second step of crystallizing the compound of Formula 2 obtained in the first step by using a mixed solvent of acetone and ethyl acetate: 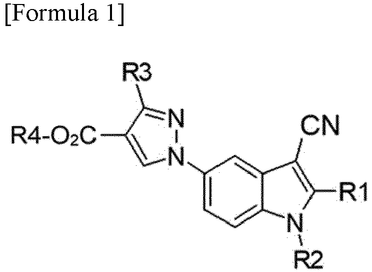 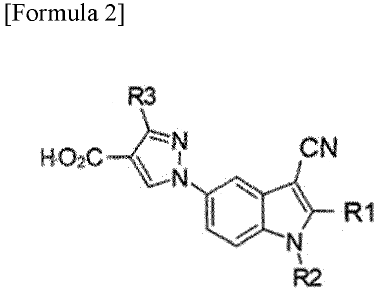
wherein,
R1 is hydrogen, halogen, C₁-C₇ alkyl, C₁-C₇ alkoxy-C₁-C₇ alkyl or phenyl;
R2 is hydrogen; C₁-C₇ alkyl unsubstituted or substituted by one or more substituents selected from halogen, C₃-C₇ cycloalkyl and O-R6; C₃-C₇ cycloalkyl; or 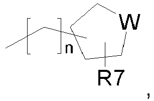 wherein R6 is C₁-C₄ alkyl, W is O or S, R7 is hydrogen or C₁-C₄ alkyl, and n is an integer of 0 to 3;
R3 is hydrogen, halogen or C₁-C₇ alkyl; and
R4 is C₁-C₇ alkyl or C₃-C₇ cycloalkyl.

2. The method according to Claim 1, wherein R2 is unsubstituted C₁-C₇ alkyl.

3. The method according to Claim 2, wherein R2 is isopropyl.

4. The method according to Claim 1, wherein the protic solvent is one or more selected from the group consisting of methanol, ethanol, propanol, butanol and acetic acid.

5. The method according to Claim 4, wherein the protic solvent is methanol.

6. The method according to Claim 1, wherein the NaOH of the first step is used at a concentration of 8 N to 12 N.

7. The method according to Claim 1, wherein the first step further comprises a step of crystallization using HCl and ethyl acetate.

8. The method according to Claim 7, wherein the HCl is used at a concentration of 2 N to 12 N.

9. The method according to Claim 1, wherein the second step further comprises a step of adding NaOH dropwise, filtrating and adding HCl dropwise to a filtrate.

10. The method according to Claim 9, wherein the HCl is added dropwise at a temperature of 48°C to 62°C.

11. The method according to Claim 9, wherein the HCl is added dropwise for 3 hours to 5 hours.
